# EUROPEAN PATENT SPECIFICATION

(11) **EP 1 676 605 B1**
(45) Date of publication and mention of the grant of the patent: **26.01.2011**
(21) Application number: 04030562.5
(22) Date of filing: 23.12.2004
(51) Int. Cl.: A61Q 5/10, B65D 47/20, A61K 8/41, A61K 8/46

(54) **Product for hair dyeing**
Haarfärbeprodukt
Produit pour la teinture des cheveux

(43) Date of publication of application: 05.07.2006
(73) Proprietor: KPSS-Kao Professional Salon Services GmbH, 64297 Darmstadt (DE)
(72) Inventor: Zeiter, Frank, 64287 Darmstadt (DE)

(56) References cited:
- EP-A- 0 391 662
- EP-A- 0 788 831
- FR-A- 2 785 878
- US-A- 4 561 570
- US-A- 5 651 793
- US-A1- 2002 144 358
- US-A1- 2003 106 910
- US-A1- 2004 244 126
- US-H1- H2 027

## Description

This invention relates to a product for oxidative dyeing hair consisting of a packaging and an aqueous composition comprising at least one oxidative dye precursor and optionally at least one coupling agent and optionally at least one direct dye which is mixed with an aqueous composition comprising at least one oxidizing agent prior to application.

Hair dyeing compositions have been marketed for many decades. Especially the oxidative hair dyeing is believed to be producing the most long lasting colours as small uncoloured molecules polymerise partly on hair surface but more importantly inside of hair so that they cannot easily been washed away with subsequent hair treatments other than colourations such as shampooing and/or conditioning hair.

These products are usually packed in single dose units because of their sensibility to oxidation when contacting air. Once packaging is opened and emptied partly, remaining product is not anymore stable. This brings certainly high packaging costs and waste, which, in addition, brings about environmental problems.

In order to solve these problems, there have been products available for oxidative hair dyeing packed into two chamber pressurized can which allow partial withdrawal of its content and the remaining part can stably be stored further (for example DE 28 27 610). This type of packaging is first of all not economic from the can material point of view (mostly aluminium) and secondly flammable gas is usually used for building up pressure inside the can between the product and outer can wall. These points bring about other disadvantages such as labelling as flammable and, therefore, special requirements in storage and transportation.

It is highly desirable to achieve a product, especially from the view point of making available multi-portion products, with low cost without loosing the performance in terms of stability and colouring ability during whole life time.

This invention starts form the problems as explained above and aims primarily for finding an alternative, which is more cost effective and environmental friendly. The alternative packaging makes withdrawal of the package content in smaller portions than the whole content of the product without affecting the stability of the remaining product during its lifetime. Lifetime of a cosmetic product in general 3 years after production.

The inventors have surprisingly found out the use of a twist cap (A) on a tube (7) or on a pouch packaging component (8) or on a bottle consisting of a seal (1), temper evident (2), thread (3) and a carrying element (4) and confectioned with a silicone valve (5) and a retainer therefore (6) filled with a hair dyeing composition comprising at least one oxidative dye precursor and optionally at least one coupling agent and optionally at least one direct dye which is mixed with an aqueous composition comprising at least one oxidizing agent prior to application, solves the above mentioned problems. Packaging with such kind of closure cap have been proposed in a US patent application published US 2003/0106910 A1.

Accordingly the object of the present invention is multiportion product for hair dyeing consisting of a packaging and an aqueous composition wherein packaging is consisting a twist cap (A) on a tube (7) or on a pouch packaging component (8) or on a bottle consisting of a seal (1), temper evident (2), thread (3) and a carrying element (4) and confectioned with a silicone valve (5) and a retainer therefore (6) filled a hair dyeing composition comprising at least one oxidative dye precursor and optionally at least one coupling agent and optionally at least one direct dye which is mixed with an aqueous composition comprising at least one oxidizing agent prior to application. The packaging as described above makes storage of an oxidative colouring composition for a long period of time possible. When the content of such packaging is only partially emptied, the remaining is as well stored without showing any signs of instability for a long time. Such kind of packaging materials are commercially available.

In the preferred from of the invention hair dyeing composition filled into the packaging mentioned above comprises at least one oxidative dye precursor and at least one coupling agent and optionally at least one direct dye which is mixed with an aqueous composition comprising at least one oxidizing agent prior to application.

In still another preferred from of the invention hair dyeing composition filled into the packaging mentioned above comprises at least one oxidative dye precursor and at least one coupling agent and at least one direct dye which is mixed with an aqueous composition comprising at least one oxidizing agent prior to application.

Further object of the present invention is multiportion product for hair dyeing consisting of a packaging and an aqueous composition wherein packaging is consisting a twist cap (A) on a tube (7) or on a pouch packaging component (8) or on a bottle consisting of a seal (1), temper evident (2), thread (3) and a carrying element (4) and confectioned with a silicone valve (5) and a retainer therefore (6) filled a hair dyeing composition comprising at least one oxidative dye precursor and optionally at least one coupling agent and optionally at least one direct dye which is mixed with an aqueous composition comprising at least one oxidizing agent prior to application
wherein the composition can be removed partially without effecting the stability of the remaining product.

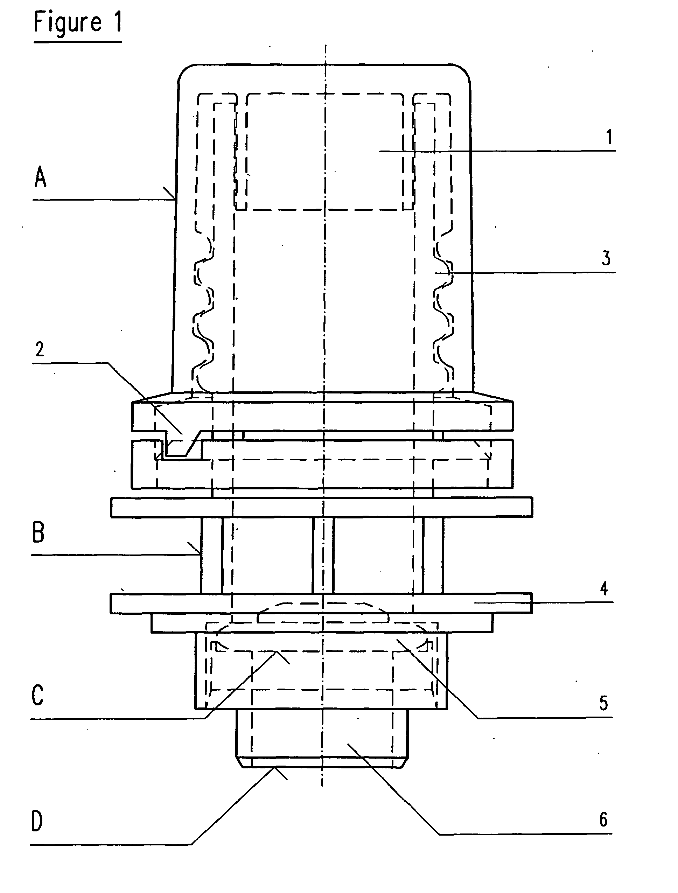

Figure 1 presents schematically a confectioned cap (The letters B, C and D all together are defined as spout.)

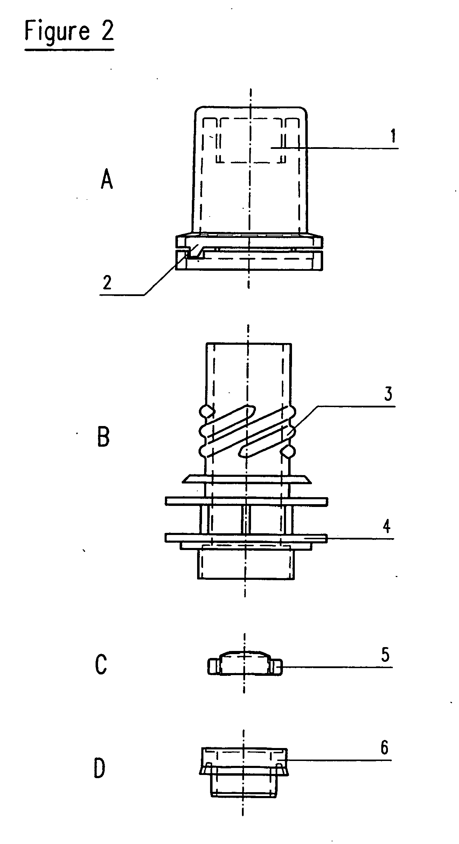

Figure 2 presents schematically individual elements of cap (The letters B, C and D all together are defined as spout.)

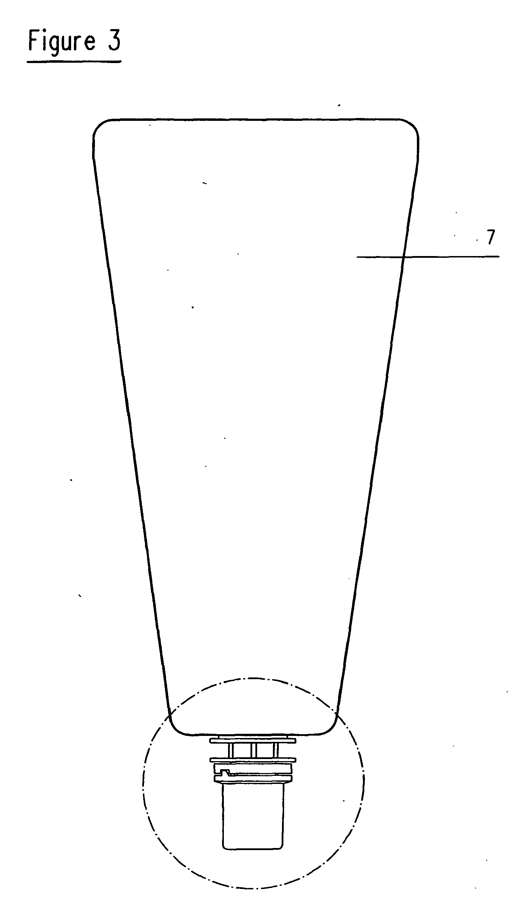

Figure 3 presents schematically a tube confectioned with a cap (circled) as shown in figures 1 and 2

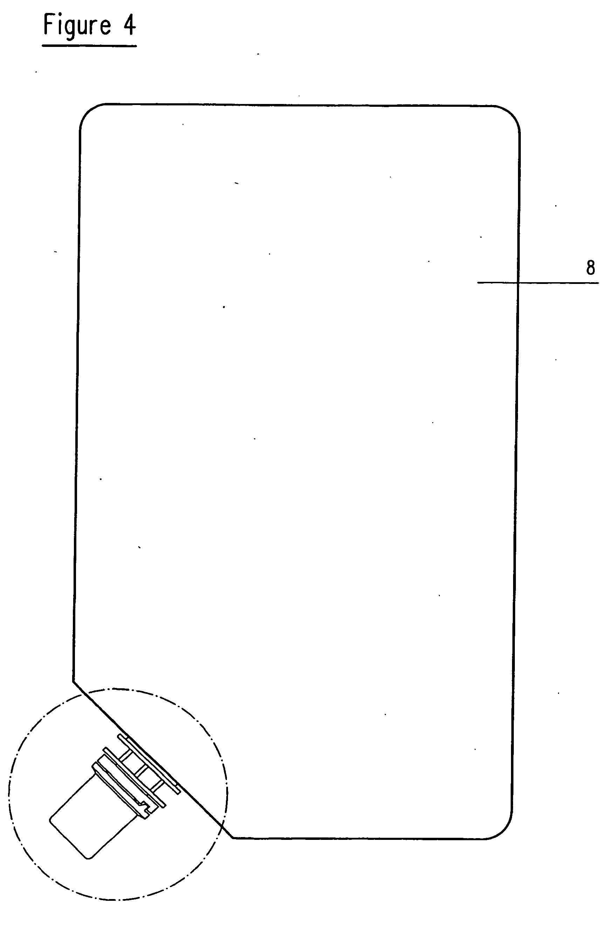

Figure 4 presents schematically a pouch confectioned with a cap (circled) as shown in figures 1 and 2

The silicone valve (5) is of heat curable liquid silicone rubber. Liquid silicone rubber used therefore is for example Silastic 94-595 from Dow Corning.

Composition filled into tube, pouch or bottle is an oxidative dyestuff composition comprising at least one oxidative dyestuff precursor (developing substance) and optionally at least one coupling substance and optionally at least one direct dye.

According to the invention, composition can be in the form of emulsion, solution, dispersion and/or gel. Emulsion and gel forms are preferred. The most preferred is emulsion.

The viscosity of the compositions according to the invention ranges from 1,000 to 50,000, in particular 1,000 to 30,000, especially 1,000 to 20,000 mPa.s, measured at 20°C with a Brookfield rotation viscosimeter, with spindle 5 at 5 rpm.

Dyeing composition according to the present invention comprises at least one oxidation dyestuff precursor (developing substance) and optionally at least one coupling substance and optionally at least one direct dye. In principal, it is possible to incorporate developing substances known per se. Special mention is made of p-phenlynediamine, p-methylaminophenol and substituted p-phenylenediamines such as 2,5-diamino-toluene, 2-n-propyl or 2-ethyl-p-phenylenediamine, 2,6-di-methyl-p-phenylene- diamine, 2-(2,5-diaminophenyl) ethanol, 1-amino-4-bis-(2'-hydroxyethyl)amino- benzene, 2-(2-hydroxyethyl amino)-5-aminotoluene, 4,4'-diaminodiphenylamine, 4-aminodiphenylamine, 2-amino-5-N,N-diethyl aminotoluene, 4-amino-N-ethyl-N-isopropyl aniline, 2-chloro-p-phenylenediamine, 1-β-hydroxyethyi-2,5-diamino-4-chlorobenzene, 1-β-hydroxyethyl-2,5-diamino-4-methyl benzene, 2-methoxy-p-phenylenediamine, N,N-diethyl-p-phenylenediamine, 1-amino-4-β-methoxyethyl aminobenzene, 1-dimethyl-amino-4-aminobenzene, 1-hydroxy-2,5-diamino-4-methyl benzene, 1-hydroxymethyl-2,5-diaminobenzene, 1,3-dimethyl-2,5-diaminobenzene, 1,4-diamino isopropyl benzene and/or 1-amino-4-β-hydroxypropyl aminobenzene, pyrazole and the derivatives thereof such as 1-hydroxyethyl-4,5-diaminopyrazole, 3,4-diamino-5-hydroxypyrazole, 3,5-diaminopyrazole, 3,5-diamino pyrazol-1-carboxamide, 3-amino-5-hydroxypyrazole, 1-phenyl-2-methylpyrazole, 1-phenyl-3-methylpyrazole-5-one, 3,5-dimethylpyrazole, 3,5-dimethylpyrazole-1-methanol, 3,5-diamino-1,2,4-triazole, 4-aminophenol and the derivatives thereof such as 4-amino-3-methylphenol, 2-chloro-4-aminophenol, 2,6-dichloro-4-aminophenol, 2,4-diamino-phenol, 2,6-dibromo-4-aminophenol, tetraamino pyrimidines, triaminohydroxy pyrimidines, diaminomono- and -dihydroxy pyrimidines, aminotriazines, 5-amino salicylic acid and/or 1,2,4-triamino benzene or the water-soluble salts thereof.

Further suitable aminopyridines are 2,5-diaminopyridine, 2,3-diaminopyridine, 2,6-diaminopyridine, 3-amino-2-methyl amino-6-methoxypyridine, 2-dimethyl-5-aminopyridine, 2-dimethyl aminoethyl-3-hydroxypyridine, 2-amino-4,6-dimethyl pyridine, 2-amino-3-hydroxypyridine, 3-amino-2(β-hydroxyethyl amino)-6-methoxypyridine, 2,6-dimethyl amino-5-aminopyridine, 2-di(hydroxyethyl) amino-5-aminopyridine, 2-hydroxyethyl amino-5-aminopyridine, 4-hydroxy-2,5,6-triaminopyrimidine and/or the water-soluble salts thereof.

The total concentration of the developing substances customarily ranges between about 0.05 % and 5 %, preferably 0.1 % and 4 %, in particular 0.25 % to 3 % by weight, calculated to the total hair dyeing composition (excluding the oxidation agent), whereby these figures are always related to the proportion of free base.

The composition according to the present invention contains optionally at least one coupling substance, which can be selected from 5-amino-2-methylphenol, 2-methyl-5-hydroxyethylaminophenol, 2,4,-diamnophenoxyehanol, 2-amino-4-hydroxyethylaminoanisol, 2-methyl-5-amino-6-chlorphenol, 1,3-bis(2,4-diaminophenoxy)propane, 2-bis(2-hydroxyethyl)aminotoluene, 2-amino-5-methylphenol, resorcinol, 2-methyl resorcinol, 4-chlororesorcinol, 2-amino-4-chlorophenol, 5-amino-4-methoxy-2-methylphenol, 2-aminophenol, 3-amino-phenol, 1-methyl-2-hydroxy-4-aminobenzene, 3-N,N-dimethyl aminophenol, 2,6-dihydroxy-3,5-dimethoxypyridine, 5-amino-3-methylphenol, 6-amino-3-methylphenol, 1,3-diamino- benzene, 1-amino-3-(2'-hydroxyethylamino)benzene, 1-amino-3-[bis(2'-hydroxy-ethyl) amino]benzene, α-naphthol, 4,6-dichlororesorcinol, 1,3-diamino-toluene, 4-hydroxy-1,2-methylenedioxy benzene, 1,5-dihydroxy naphthalene, 1,6-dihydroxy naphthalene, 1,7-dihydroxy naphthalene, 2,7-dihydroxy naphthalene, 1-hydroxy-2-methyl naphthalene, 4-hydroxy-1,2-methyldioxy benzene, 2,4-diamino-3-chlorophenol, 5-amino-2-methoxyphenol and/or 1-methoxy-2-amino-4-(2'-hydroxyethyl amino)benzene or the water-soluble salts thereof.

Further, indole and indoline derivatives can as well be contained in the colouring composition of the present invention. Examples to those are: 6-aminoindol, 6-hydroxyindole, 1-ethyl-6-hydroxyindole, 1-methyl-4-hydroxyindol, 1-methyl-6-hydroxyindole, 2-methyl-6-hydroxyindole, 5-hydroxyindol, 4-hydroxyindol, 5,6-dihydroxyindole, 6-aminoindoline, 6-hydroxyindoline, 1-ethyl-6-hydroxyindoline, 1-methyl-4-hydroxyindoline, 1-methyl-6-hydroxyindoline, 2-methyl-6-hydroxyindoline, 5-hydroxyindoline, 4-hydroxyindoline, 5,6-dihydroxyindoline and their respective salts.

In the hair dyeing compositions according to the invention, the coupling substance(s) as reaction partners of the developing substance(s) are present in approximately the same molecular proportions as the developing substances, i.e. in amounts from 0.01 % to 5.0 %, preferably 0.05 % to 4 %, in particular 0.1 % to 3 % by weight, calculated to the total composition (excluding the oxidizing agent), whereby these figures are always related to the proportion of free base.

According to the invention, dyeing composition comprises optionally at least one direct acting dyestuff selected from anionic, cationic and neutral ones. Suitable cationic dyestuffs are in principal those available on the market for hair colouring applications. Some examples to those are:
Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 76, Basic Violet 1, Basic Violet 2, Basic Violet 3, Basic Violet 10, Basic Violet 14 and Basic Yellow 57.

For this purpose, special reference is made to the PCT application WO 95/15144 of Ciba-Geigy AG. The cationic dyestuffs with the following chemical structures are especially, the most, preferred ones according to the present invention. 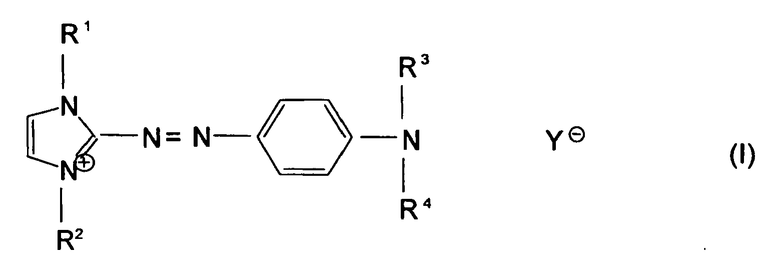 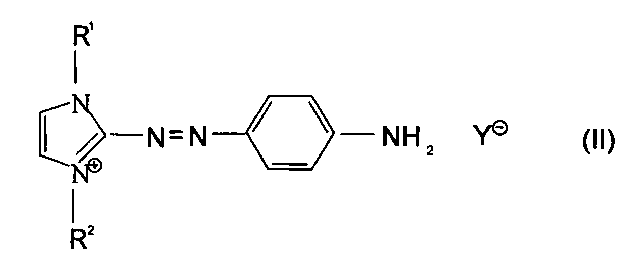 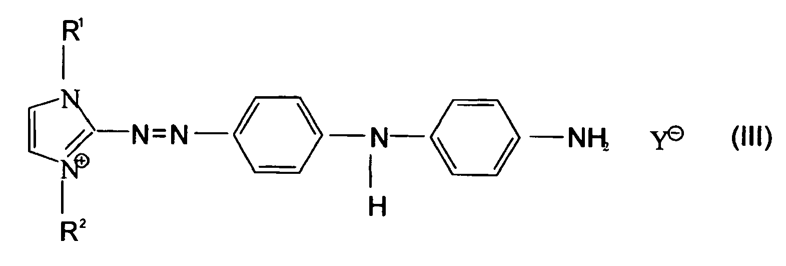 and 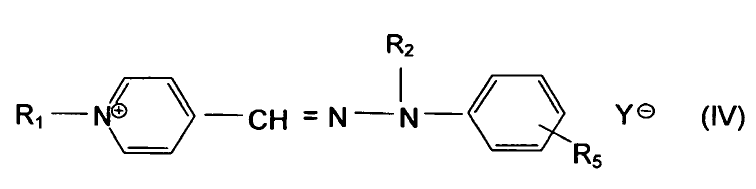 wherein R¹, R², R³ and R⁴ stand for hydrogen, a CH₃- or C₂H₅- group, R⁵ stands for hydrogen, -OCH₃ or -OC₂H₅ and Y is an anion such as chloride, bromide, methosulfate.

The most preferred compounds are the ones according to the formula I, where R₁, R₂, R₃ and R₄ are methyl and Y is chloride, according to formula II where R₁ and R₂ are methyl and Y is chloride and according to the formula IV, where R₁ and R₂, are methyl, R₅ is hydrogen and Y is methosulfate.

Cationic dyestuffs are included into the compositions of the present invention at a concentration of 0.01 to 7.5%, preferably 0.05 to 5% and more preferably 0.05 to 3% by weight calculated to total aqueous composition.

### Anionic dyes suitable for the compositions of the present invention are:

Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium.

Among those, the most preferred anionic dyestuffs are Acid Red 52, DC Violet 2, DC Red 33, DC Orange 4, DC Red 27 and DC Yellow 10.

According to the invention anionic dyes are included into oxidative dyeing compositions at a concentration of 0.1 to 7.5%, preferably 0.1 to 5%, more preferably 0.1 to 3% by weight calculated to total aqueous composition.

When both anionic and cationic dyes are present in the dyeing compositions the ratio of cationic dyestuffs to acidic dyestuffs by weight is preferably in the range of 3:1 to 1:10, preferably 2: 1 to 1:7 and further more preferably 2:1 to 1:5. In a patent application from the applicant the importance of the ratio of cationic to anionic dyes is disclosed in detail.

Additionally, the coloring compositions of the present invention can comprise neutral nitro dyes (HC dyes), so called nitro dyes as direct dye. Concentration of those can typically be in the range from 0.01 to 2.5%, preferably 0.1 to 2% by weight calculated to total aqueous composition.

Some examples to those are: HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Plant dyestuffs can also be used alone or in combination with synthetic direct-acting dyestuffs, for example henna (red or black), alkanna root, laccaic acid, indigo, logwood powder, madder root and rhubarb powder, etc.

pH of the aqueous composition of the present invention varies between 5 and 12, preferably 6 - 11, more preferably 6.8 to 10. pH of the aqueous composition is adjusted to the required pH by using triethanolamine, ammonia or its salts with acids such as ammonium chloride, ammonium sulphate, ammonium carbonate, ammonium bicarbonate, ammonium nitrate, or using alkaline solutions such as sodium hydroxide, potassium hydroxide and their respective salts with the known acids.

In the case that the composition is an emulsion, the most preferred, aqueous composition for oxidative dyeing comprises in the base formulation fatty acids with 0 to 3 ethylenic bonds and with fatty acyl chain length of 12 to 22 C atom. Concentration of the fatty acids can be in the range of 0.1 to 10%, preferably 0.1 to 7.5% and most preferably 0.2 to 5% by weight calculated to the total composition. Fatty acid examples, without limiting the choice, suitable for colouring compositions are myristic acid, palmitic acid, behenic acid, steraic acid, oleic acid, linoleic acid. The most preferred fatty acid is oleic acid.

In the case that the coloring composition is in the form of an emulsion, it comprises as an emulsion base at least one fatty alcohol or mixture of fatty alcohols with the chain length of 14 to 22 C atoms. Examples to suitable fatty alcohols, without limiting the choice, are myristyl alcohol, cetyl alcohol, stearyl alcohol, behenyl alcohol and cetostearyl alcohol. The most preferred is cetostearyl alcohol well known with its trade name Lanette O or as Lanette N in mixture with sodium cetearyl sulfate from Cognis.

The concentration of fatty alcohol(s) is in the range from 0.5 to 25%, preferably 0.5 to 20% by weight and more preferably 0.5 to 15% by weight calculated to total composition without oxidizing agent.

Coloring compositions according to present invention comprises surfactants selected from anionic, nonionic, amphoteric (or zwiterionic) and/or cationic surfactants as emulsifier or solubilizer. Cationic surfactants are as well used as hair conditioners in the coloring composition.

Anionic surfactants suitable within the scope of the invention are in principal known from the cleansing compositions and are preferably present in an amount from 0.1 to about 10%, preferably 0.2 to 7.5% and most preferably 0.2 - 5% by weight, calculated to the total composition.

These are anionic surfactants of the sulfate, sulfonate, carboxylate and alkyl phosphate type, for example, the known C₁₀-C₁₈-alkyl sulfates, and in particular the respective ether sulfates, for example, C₁₂-C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates.

Additional anionic surfactants useful within the scope of the invention are α-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof of the formula

R₆- (C₂H₄O)ₙ- O - CH₂COOX,

wherein R₆ is a C₈-C₂₀-alkyl group, preferably a C₁₂-C₁₄-alkyl group, n is a number from 1 to 20, preferably 2 to 17, and X is H or preferably a cation of the group sodium, potassium, magnesium and ammonium, which can optionally be hydroxyalkyl-substituted, as well as alkyl amido polyether carboxylic acids of the general formula 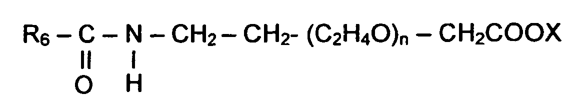 wherein R₆ and X have the above meanings, and n is in particular a number from 1 to 10, preferably 2.5 to 5.

Such products have been known for some time and are on the market, for example, under the trade name "AKYPO® " and "AKYPO-SOFT® ".

Also useful are C₈-C₂₀-acyl isethionates, alone or in admixture with other anionic surfactants, as well as sulfofatty acids and the esters thereof.

Further suitable anionic surfactants are also C₈-C₂₂-acyl aminocarboxylic acids or the water-soluble salts thereof. Especially preferred is N-lauroyl glutamate, in particular as sodium salt, as well as, for example, N-lauroyl sarcosinate, N-C₁₂-C₁₈-acyl asparaginic acid, N-myristoyl sarcosinate, N-oleoyl sarcosinate, N-lauroyl methylalanine, N-lauroyl lysine and N-lauroyl aminopropyl glycine, preferably in form of the water-soluble alkali or ammonium, in particular the sodium salts thereof, preferably in admixture with the above-named anionic surfactants.

It is also possible to use mixtures of several anionic surfactants in a mixture.

An overview of the anionic surfactants suitable for the present invention can furthermore be found in the monography of K. Schrader, "Grundlagen und Rezepturen der Kosmetika", 2nd Ed.(1989, Hüthig Buchverlag), pp. 595-600 and pp. 683 to 691.

Further surfactants in the colouring compositions according to the invention are nonionic surfactants alone or in admixture with anionic surfactants at a weight ratio of 3:1 to 1:3.

These are described as well in Schrader, I.c., on pages 600-601 and pp. 694-695.

Especially suited nonionic surfactants are, for example, long-chain fatty acid mono-and dialkanolamides, such as coco fatty acid mono- or diethanolamide and myristic fatty acid mono or diethanolamide, stearic acid mono or diethanolamide.

Further nonionic surfactants suited are alkyl polyglucosides of the general formula

R₇-O-(R₃O)ₙ-Zₓ,

wherein R₇ is an alkyl group with 8 to 18 carbon atoms, R₃ is an ethylene or propylene group, Z is a saccharide group with 5 to 6 carbon atoms, n is a number from 0 to 10 and x is a number between 1 and 5.

Further additionally useful nonionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics^{R}", as well as fatty alcohol ethoxylates.

Further suitable nonionic surfactants are amineoxides. Such amineoxides are state of the art, for example C₁₂-C₁₈- alkyl dimethyl amineoxides such as lauryl dimethyl amineoxide, C₁₂-C₁₈- alkyl amidopropyl or -ethyl amineoxides, C₁₂-C₁₈ -alkyl di(hydroxyethyl) or (hydroxypropyl) amineoxides, or also amineoxides with ethyleneoxide and/or propyleneoxide groups in the alkyl chain. Such amineoxides are on the market, for example, under the trade names "Ammonyx® ", "Aromox® " or "Genaminox® ".

Further nonionic surfactants useful in the compositions according to invention are C₁₀-C₂₂-fatty alcohol ethoxylates. Especially suited are C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16":
The average degree of ethoxylation thereby ranges between about 2.5 and about 25, preferably about 10 and about 20.

The concentration of nonionic surfactant is in the range from 0.5 to 15%, preferably 0.5 to 10% by weight and more preferably 0.5 to 7.5% by weight calculated to total composition without oxidizing agent.

As further surfactant component, the colouring compositions according to the invention can also contain amphoteric or zwitterionic surfactants, for example in an amount from about 0.5 % to about 5 %, preferably from about 1 % to about 2.5 % by weight, calculated to the total composition without oxidizing agent.

Useful as such are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate have also proven suitable.

In detail, it is possible to use betaines of the structures 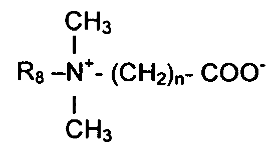 and 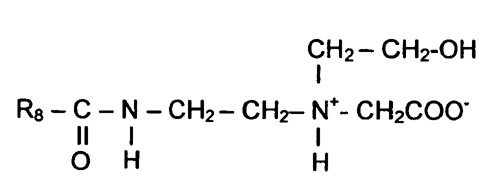 wherein R₈ is a C₈-C₁₈-alkyl group and n is 1 to 3; sulfobetaines of the structure 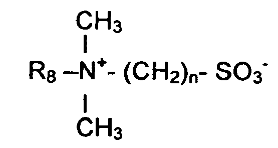 wherein R₈ and n are same as above; and amidoalkyl betaines of the structure 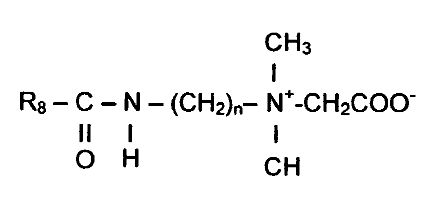 wherein R₈ and n are same as above.

Colouring composition can contain cationic surfactants as emulsifier, solubilizer and/or conditioning ingredients according to the formula, but not limited to 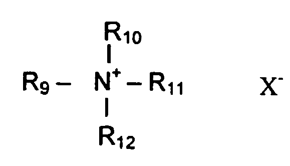 where R₉ is a saturated or unsaturated, branched or non-branched alkyl chain with 8-22 C atoms or

R₁₃CO NH (CH₂)ₙ

where R₁₃ is saturated or unsaturated, branched or non-branched alkyl chain with 7-21 C atoms and n has typical value of 1 - 4 or

R₁₄ CO O (CH₂)ₙ

where R₁₄ is saturated or unsaturated, branched or non-branched alkyl chain with 7-21 C atoms and n has typical value of 1 - 4, and
R₁₀ is H or unsaturated or saturated, branched or non-branched alkyl chain with 1 - 22 C atoms or

R₁₃ CO NH (CH₂)ₙ

or

R₁₄ CO O (CH₂)ₙ

where R₁₃, R₁₄ and n are same as above.

R₁₁ and R₁₂ are H or lower alkyl chain with 1 to 4 Carbon atoms, and X is typically an anion such as chloride, bromide, methosulfate.

Typical examples of those ingredients are cetyl trimethyl ammonium chloride, steartrimonium chloride, dipalmitoyl dimonium chloride, distearyl dimethyl ammonium chloride, stearamidopropyl trimonuim chloride, dioleoylethyl dimethyl ammonium methosulfate, dioleoylethyl hydroxyethylmonium methosulfate.

Colouring composition can also contain cationic polymers as conditioning agents and gel forming agents. Those are cationic cellulose type polymers know as Polymer JR type from Amerchol such as Polyquaternium 10 or cationic guar gum known with trade name Jaguar from Rhône-Poulenc and chemically for example Guar hydroxypropyl trimonium chloride. Furthermore, chitosan and chitin can also be included in the compositions as cationic natural polymers.

Furthermore, it has been found suitable those cationic polymers known with their CTFA category name Polyquaternium. Typical examples of those Polyquaternium 6, Polyquaternium 7, Polyquaternium 10, Polyquaternium 11, Polyquaternium 16, Polyquaternium 22 and Polyquaternium 28, Polyquaternium 30, Polyquaternium 37, Polyquaternium 36, Polyquaternium 46. Among those the most preferred one is the Polyquaternium 11 as well known with its trade name Gafquat from ISP and as Luviquat PQ from BASF.

As well those polymers known with their CTFA category name Quaternium are suitable. Those are for example Quaternium-8, Quaternium-14, Quaternium-15, Quaternium-18, Quaternium-22, Quaternium-24, Quaternium-26, Quaternium-27, Quaternium-30, Quaternium-33, Quaternium-53, Quaternium-60, Quaternium-61, Quaternium-72, Quaternium-78, Quaternium-80, Quaternium-81, Quaternium-81, Quaternium-82, Quaternium-83 and Quaternium-84.

Typical concentration range for any of the cationic conditioners mentioned above can be 0.01 - 5% by weight, preferably 0.05 - 3% by weight and more preferably 0.05 - 2% by weight calculated to total composition without oxidizing agent.

Colouring composition comprise preferably an organopolysiloxane wherein at least one silicium atom is linked to an alkylene group having a hetero-atom, in particular a nitrogen atom, with a poly-(N-acyl alkyleneimine) units of the formula 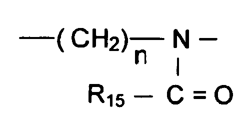 wherein n is a number from 1 to 5 and R₁₅ is hydrogen, a C₁-C₁₂-alkyl or cycloalkyl, aralkyl or aryl group.

Preferred organopolysiloxane polymers are those of the type disclosed in EP-A 640 643, in particular optionally quaternized aminoalkyl, in particular aminopropyl dimethyl polysiloxane/polyethyl oxazoline copolymers of the formula 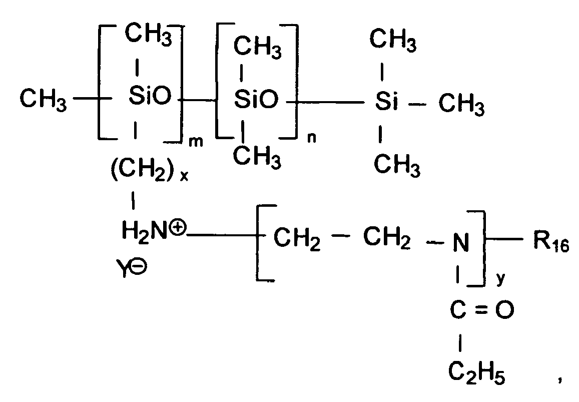 wherein m and n each are numbers from 20 to 10,000, in particular 50 to 7,000, especially 100 to 5,000, x is a number between 1 and 5, preferably 3, and y is a number from 5 to 30, R₁₆ is a C₁-C₁₂-alkyl or aryl group, in particular a methyl, ethyl or benzyl group, and Y- is an anion.

Especially suited are the organopolysiloxanes disclosed under the terms A-1, A-2 and A-3 on pages 12 to 13 of EP-A 640 643. The proportion of graft copolymers in the hair colouring compositions according to the invention ranges from 0.05 % to 5 %, preferably 0.1 % to 2.5 %, in particular 0.5 % to 1.5 % by weight, calculated to the total composition.

Colouring compositions according to the present invention can contain organic solvents as penetration enhancers and also as a solubilzers. Examples of such organic solvents are benzyloxy ethanol, benzyl alcohol, phenoxy ethanol, phenoxy isopropanol, methyl phenoxy ethanol, benzyl glycerol, N-benzyl formide, N-methyl pyrrolidone, N-ethyl pyrrolidone, cinnamyl alcohol, phenethyl alcohol, p-methyl benzyl alcohol, butyl cellosolve, methyl carbitol, ethyl carbitol, propyl carbitol, butyl carbitol, diethyleneglycol, diethyl ether and dipropyleneglycol diethyl ether. Typically the concentration of those solvents can be in the range from 0.5% to 25%, preferably 0.5 - 20% by weight and more preferably 0.5 -15% by weight calculated to the total composition without oxidizing agent.

The hair dyeing compositions according to the invention preferably contain thickening agents, especially in the case a gel type of preparation is preferred. These are, for example, the various cellulose derivatives such as hydroxyalkyl celluloses, e.g. hydroxyethyl cellulose, hydroxypropyl cellulose, hydroxypropyl methyl cellulose, natural polysaccharides such as xanthan gum; guar gum and the alkoxylation products thereof in amounts from 0.1 - 5 %, preferably 0.1 - 3% and most preferably 0.1 - 2% by weight calculated to the total composition and depending on the desired consistency thereof.

Optionally, the composition of this invention can comprise further hair conditioning agents such as silicone oils either volatile or non-volatile, natural and synthetic oils. Among silicone oils those can be added to the colouring composition include dimethicone, dimethiconol, polydimethylsiloxane, DC fluid ranges from Dow Corning, natural oils such as olive oil, almond oil, avocado oil, weizenkeim oil, ricinus oil and the synthetic oils, such as mineral oil, isopropyl myristate, palmitate, stearate and isostearate, oleyl oleate, isocetyl stearate, hexyl laurate, dibutyl adipate, dioctyl adipate, myristyl myristate and oleyl erucate.

Additional non-ionic conditioning agents may be polyols such as glycerin, glycol and derivatives, polyethyleneglycoles known with trade names Carbowax PEG from Union Carbide and Polyox WSR range from Amerchol, polyglycerin, polyethyleneglycol mono or di fatty acid esters having general formula X or XI, respectively,

R₁₇ CO (O CH₂ CH₂)ₙ OH formula X

R₁₇ CO (O CH₂ CH₂)ₙ O OC R₁₈ formula X

where R₁₇ and R₁₈ are independent from each other saturated, unsaturated or branched or non-branched alkyl chain with 7 to 21 C atoms and n is typically 2 - 100.

Another preferred compound in the composition is of ceramide type of compounds according to general formula 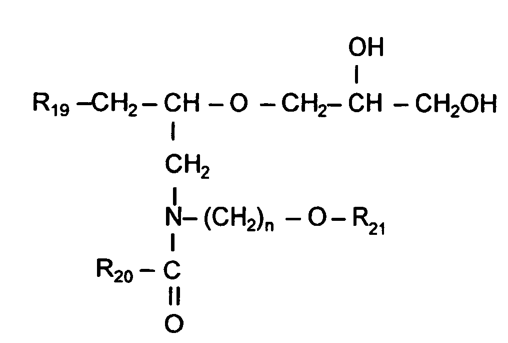 where R₁₉ and R₂₀ are independent from each other alkyl- or. alkenyl group mit 10 to 22 carbon atoms, R₂₁ is methyl, ethyl, n-propyl or isopropyl group and n is a number between 1 to 6, preferably 2 or 3. The concentration of ceramide type of compound in colouring compositions of the present invention can be in the range of 0.01 to 2 and especially 0.01 to 1% by weight calculated to the total composition.

The compositions of the present invention can comprise of at least one ubiquinone of the formula (I) 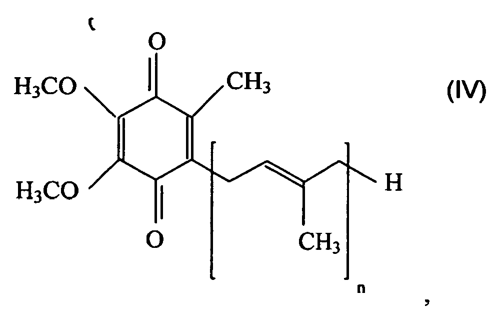 wherein n is a number from 1 to 10. Concentration of ubichinone can vary between 0.001 % and 10 % by weight, calculated to the total composition, excluding the oxidizing agent.

After removal of the content of the product comprising at least one oxidation dyestuff precursor and optionally at least one coupling substance and optionally at least one direct dye, the composition is mixed with an aqueous composition comprising at least one oxidizing agent prior to application onto hair. The oxidizing agents suitable are hydrogen peroxide, urea peroxide, melanin peroxide or perborate salts. The most preferred is hydrogen peroxide, which is used as a lotion containing 3 to 12% by weight, calculated to composition only comprising hydrogen peroxide. The mixing ratio is usually in the range of 4:1 to 1:4, by weight, preferably 3:1 to 1:3 by weight, more preferably 2:1 to 1:3 by weight.

The tubes or pouches or bottles can be emptied with very low level of remains in the packaging. The emptying is achieved at most not les than 98% of its content by weight.

Filling of the aqueous composition into tube or pouch or bottle for obtaining product for oxidative colouring hair comprising at least one oxidation dyestuff precursor and optionally at least one coupling substance and optionally at least one direct dye is carried out in a process in which the compositions is filled into the tube or pouch or bottle with an extendable filling nozzle entering into pouch or tube or bottle in order to secure the long lifetime of the content - long-term stability.

The following examples are used to illustrate the invention, but not limit it

### Examples

**Base formulations**

| | % by weight | % by weight |
|---|---|---|
| | I | II |
| Stearamide MEA | - | 1.50 |
| Cocamide MEA | 4.00 | 2.00 |
| Cetearyl alcohol | 10.00 | 8.00 |
| Tegin P | 1.40 | 1.40 |
| Propylene Glycol | 2.40 | 1.60 |
| Oleic acid | 3.00 | 3.00 |
| Ammonium chloride | 0.50 | 0.50 |
| Tetrasodium EDTA | 0.20 | 0.20 |
| Sodium lauryl sulfate | 1.50 | - |
| Sodium cetearyl sulfate | - | 1.20 |
| Organopolysiloxane A1 of EP 640 643 | 0.20 | 0.20 |
| Ceramide according to formula wherein R₁₈ and R₁₉ are C₁₆ alkyl and R₂₀ is ethyl | 0.10 | 0.20 |
| | | |
| Water | to 100 | to 100 |

The dyestuffs given in the following as for various color directions are added to the base compositions either I or II. Water amount is reduced in the base formula corresponding to the amount of dyes present in the formulations.
The both base formulations have similar viscosity values of around 5000 mPa s measured by Brookfield viscosimeter at 20°C with spindle 4.

### Example 1

| | % by weight |
|---|---|
| p-Toluenediamine sulfate | 1.40 |
| Resorcin | 0.60 |
| 3-Aminophenol | 0.10 |
| 2-Amino-4-hydroxyethylaminoanisolsulfate | 0.02 |
| Base formula I or II | to 100 |

The pH of the composition is adjusted to 9.5. The composition obtained was mixed with a 6% by weight hydrogen peroxide comprising composition at a weight ratio of 1:1 and hair swatches were colored at room temperature for 30 min. A light brown color was obtained.

The composition of example 1 was filled into a pouch and a tube as described above. The product was stable over the period of 12 weeks (period tested) at 40°C and at room temperature. No instability is observed in terms of colouring performance on hair, pH, viscosity, appearance (especially macroscopic colour of the composition).

In another test the same product was filled into a tube and a pouch with a volume of 100 ml according to the invention and in a tube and a pouch without containing the silicone valve, meaning that when the cap is opened the content is directly in contact with air. All products were stored at room temperature and their content was emptied every week by removing approximately 15 g. With the removed composition colouring test was carried out as described above. Comparison was also made with a freshly prepared composition. The evaluation was carried out according to following scale.
1 extremely weak colouring
2 very weak colouring
3 colouring with low intensity
4 intensive colouring
5 very intensive colouring

The results are presented in Table I.

**Table I: Results of colouring test of the products stored in different packaging as described above and a freshly prepared colouring composition.**

| Storage | Example 1 stored in according to (see above) | | |
|---|---|---|---|
| Period (weeks) | Invention* | Comparative* | Fresh composition |
| 1 | 5 | 5 | 5 |
| 2 | 5 | 4 | 5 |
| 3 | 4 | 3 | 5 |
| 4 | 5 | 1 | 5 |
| 6 | 5 | 1 | 5 |

| | | | |
|---|---|---|---|
| * either pouch or tube, no difference was observed | | | |

From the above results it is clear that the composition stored in a packaging according to invention shows very good colouring performance and therefore without oxidative polymerization of the oxidative dyes during storage, whereas the composition stored in a pouch or tube with a cap without silicone valve did not show any coloruing effect after storing 4 weeks at room temperature indicating that the oxidative dyes are not stable under those conditions. Thus, the product of the invention has a longer shelf life than that of comparative product.

Similar results are obtained with the following examples as well.

### Example 2

| | % by weight |
|---|---|
| 1-Hydroxyethyl-4,5-diaminopyrazolsulfate | 0.54 |
| 4-Amino-2-hydroxytoluol | 0.27 |
| Base formula I or II | to 100 |

The preparation and filling and colouring tests were carried out as above and Red copper colour is obtained.

### Example 3

| | % by weight |
|---|---|
| 2,4,5,6-Tetraaminopyrimidine sulphate | 0.77 |
| 2-Amino-4-hydroxyethylaminoanisolsulfate | 0.90 |
| Base formula I | to 100 |

The preparation and filling and colouring tests was carried out as above and violet colour is obtained.

### Example 4

| | % by weight |
|---|---|
| 2,4,5,6-Tetraaminopyrimidine sulphate | 0.53 |
| 2-Methylresorcin | 0.28 |
| Basic red 51 | 0.20 |
| Base formula I or II | to 100 |

The preparation and filling and colouring tests was carried out as above and intensive red colour is obtained.

### Example 5

| | % by weight |
|---|---|
| 2,4-Diamino-6-hydroxypyrimidine | 0.28 |
| 4-amino-2hydroxytoluol | 0.27 |
| Base formula I or II | to 100 |

The preparation and filling and colouring tests was carried out as above and orange colour is obtained.

## Claims

1. Multiportion product for oxidative colouring hair consisting of a packaging consisting of a twist cap (A) on a tube (7) consisting of a seal (1), temper evident (2), thread (3) and a carrying element (4) and confectioned with a silicone valve (5) and a retainer therefore (6) and an aqueous oxidative colouring composition comprising at least one oxidative dye precursor and optionally at least one coupling agent and optionally at least one direct dye which is mixed with an aqueous composition comprising at least one oxidizing agent prior to application, wherein the composition can be removed partially without affecting the stability of the remaining composition.

2. Multiportion product for oxidative clouting hair consisting of a packaging consisting of a twist cap (A) on a pouch packaging component (8) consisting of a seal (1), temper evident (2), thread (3) and a carrying element (4) and confectioned with a silicone valve (5) and a retainer therefore (6) and an aqueous oxidative colouring composition comprising at least one oxidative dye precursor and optionally at least one coupling agent and optionally at least one direct dye which is mixed with an aqueous composition comprising at least one oxidizing agent prior to application wherein the composition can be removed partially without affecting the stability of the remaining composition.

3. Multiportion product for oxidative colouring hair consisting of a packaging consisting of a twist cap (A) on a bottle consisting of a seal (1), temper evident (2), thread (3) and a carrying element (4) and confectioned with a silicone valve (5) and a retainer therefore (6) and an aqueous oxidative colouring composition comprising at least one oxidative dye precursor and optionally at least one coupling agent and optionally at least one direct dye which is mixed with an aqueous composition comprising at least one oxidizing agent prior to application wherein the composition can be removed partially without affecting the stability of the remaining composition.

4. Multiportion product according to any of the preceding claims **characterised in that** the aqueous colouring composition has a viscosity in the range from 1,000 to 50,000 mPa.s measured at 20°C with a Brookfiled viscosimeter with a spindle 5 at 5 rpm.

5. Multiportion product according to any of the preceding claims **characterised in that** it comprises at least one coupling substance in addition to oxidative dye precursor.

6. Multiportion product according to any of the preceding claims **characterised in that** it comprises at least one direct dye selected from anionic, cationic and neutral nitro dyes,

7. Multiportion product according to claim 6 that the direct dye is cationic hair dye.

8. Multiportion product according to claim 6 that the direct dye is neural nitro hair dye.

9. Multiportion product according to any of the preceding claims **characterised in that** the aqueous oxidative dyeing composition is an emulsion and comprises at least one emulgator.

10. Multiportion product according to any of the preceding claims **characterised in that** the aqueous oxidative dyeing composition has a pH between 5 and 12.

11. Use of a packaging consisting of a twist cap (A) on a tube (7) consisting of a seal (1), temper evident (2), thread (3) and a carrying element (4) and confectioned with a silicone valve (5) and a retainer therefore (6) for stably storing an aqueous oxidative colouring composition comprising at least one oxidative dye precursor and optionally at least one coupling agent and optionally at least one direct dye which is mixed with an aqueous composition comprising at least one oxidizing agent prior to application wherein the composition can be removed partially without affecting the stability of the remaining composition.

12. Use of a packaging consisting of a twist cap (A) on a pouch packaging component (8) consisting of a seal (1), temper evident (2), thread (3) and a carrying element (4) and confectioned with a silicone valve (5) and a retainer therefore (6) for stably storing an aqueous oxidative colouring composition comprising at least one oxidative dye precursor and optionally at least one coupling agent and optionally at least one direct dye which is mixed with an aqueous composition comprising at least one oxidizing agent prior to application wherein the composition can be removed partially without affecting the stability of the remaining composition.

13. Use of a packaging consisting of a twist cap (A) on a bottle consisting of a seal (1), temper evident (2), thread (3) and a carrying element (4) and confectioned with a silicone valve (5) and a retainer therefore (6) for stably storing an aqueous oxidative colouring composition comprising at least one oxidative dye precursor and optionally at least one coupling agent and optionally at least one direct dye which is mixed with an aqueous composition comprising at least one oxidizing agent prior to application wherein the composition can be removed partially without affecting the stability of the remaining composition.

## Patentansprüche

1. Mehrportionen Produkt zum oxidativen Färben von Haaren, bestehend aus einer Verpackung mit einer Schraubkappe (A) an einer Tube (7) die aus einer Dichtung (1), einem Sicherheitsring (2), Gewinde (3) und einem Tragelement (4) besteht und mit einem Silikonventil (5) und einer Arretierung (6) konfektioniert ist, gefüllt mit einem wässrigen oxidativen Färbemittel welches mindestens einen oxidativen Farbentwickler und mindestens eine Kupplerverbindung und optional mindestens eine direkt ziehende Farbe enthält, die vor der Applikation mit einer wässrigen Zusammensetzung die mindestens eine oxidierende Verbindung enthält gemischt wird, wobei die Zusammensetzung in Teilen entnommen werden kann, ohne die Stabilität des verbleibenden Produkts zu beeinträchtigen.

2. Mehrportionen Produkt zum oxidativen Färben von Haaren, bestehend aus einer Verpackung mit einer Schraubkappe (A) an einem Verpackungsbeutel (8) die aus einer Dichtung (1), einem Sicherheitsring (2), Gewinde (3) und einem Tragelement (4) besteht und mit einem Silikonventil (5) und einer Arretierung (6) konfektioniert ist, gefüllt mit einem wässrigen oxidativen Färbemittel welches mindestens einen oxidativen Farbentwickler und mindestens eine Kupplerverbindung und optional mindestens eine direkt ziehende Farbe enthält, die vor der Applikation mit einer wässrigen Zusammensetzung die mindestens eine oxidierende Verbindung enthält gemischt wird, wobei die Zusammensetzung in Teilen entnommen werden kann, ohne die Stabilität des verbleibenden Produkts zu beeinträchtigen.

3. Mehrportionen Produkt zum oxidativen Färben von Haaren, bestehend aus einer Verpackung mit einer Schraubkappe (A) an einer Flasche die aus einer Dichtung (1), einem Sicherheitsring (2), Gewinde (3) und einem Tragelement (4) besteht und mit einem Silikonventil (5) und einer Arretierung (6) konfektioniert ist, gefüllt mit einem wässrigen oxidativen Färbemittel welches mindestens einen oxidativen Farbentwickler und mindestens eine Kupplerverbindung und optional mindestens eine direkt ziehende Farbe enthält, die vor der Applikation mit einer wässrigen Zusammensetzung die mindestens eine oxidierende Verbindung enthält gemischt wird, wobei die Zusammensetzung in Teilen entnommen werden kann, ohne die Stabilität des verbleibenden Produkts zu beeinträchtigen.

4. Mehrportionen Produkt nach einem der vorhergehenden Ansprüche, **dadurch gekennzeichnet, dass** das wässrige Färbemittel eine Viskosität im Bereich von 1.000 bis 50.000 mPa.s hat, gemessen bei 20°C mit einem Brookfield Viskosimeter mit einer Spindel 5 bei 5 rpm.

5. Mehrportionen Produkt nach einem der vorhergehenden Ansprüche, **dadurch gekennzeichnet, dass** es mindestens eine Kupplersubstanz zusätzlich zu einem oxidativen Farbentwickler enthält.

6. Mehrportionen Produkt nach einem der vorhergehenden Ansprüche, **dadurch gekennzeichnet, dass** es mindestens eine direkt ziehende Farbe enthält, ausgewählt aus anionischen-, kationischen- und neutralen Nitrofarbstoffen.

7. Mehrportionen Produkt nach Anspruch 6, in dem der Direktfarbstoff ein kationische Haarfarbstoff ist.

8. Mehrportionen Produkt nach Anspruch 6, in dem der Direktfarbstoff ein neutraler Nitrofarbstoff ist.

9. Mehrportionen Produkt nach einem der vorhergehenden Ansprüche, **dadurch gekennzeichnet, dass** das wässrige oxidative Färbemittel eine Emulsion ist und mindestens einen Emulgator enthält.

10. Mehrportionen Produkt nach einem der vorhergehenden Ansprüche, **dadurch gekennzeichnet, dass** das wässrige oxidative Färbemittel einen pH- Wert zwischen 5 und 12 hat.

11. Verwendung einer Verpackung, bestehend aus einer Schraubkappe (A) an einer Tube (7) die aus einer Dichtung (1), einem Sicherheitsring (2), Gewinde (3) und einem Tragelement (4) besteht und mit einem Silikonventil (5) und einer Arretierung (6) konfektioniert ist, zur stabilen Aufbewahrung eines wässrigen oxidativen Färbemittels welches mindestens einen oxidativen Farbentwickler und optional mindestens eine Kupplerverbindung und optional mindestens eine direkt ziehende Farbe enthält, die vor der Applikation mit einer wässrigen Zusammensetzung die mindestens eine oxidierende Verbindung enthält gemischt wird, wobei die Zusammensetzung in Teilen entnommen werden kann, ohne die Stabilität des verbleibenden Produkts zu beeinträchtigen.

12. Verwendung einer Verpackung, bestehend aus einer Schraubkappe (A) an einem Verpackungsbeutel (8) die aus einer Dichtung (1), einem Sicherheitsring (2), Gewinde (3) und einem Tragelement (4) besteht und mit einem Silikonventil (5) und einer Arretierung (6) konfektioniert ist, zur stabilen Aufbewahrung eines wässrigen oxidativen Färbemittels welches mindestens einen oxidativen Farbentwickler und optional mindestens eine Kupplerverbindung und optional mindestens eine direkt ziehende Farbe enthält, die vor der Applikation mit einer wässrigen Zusammensetzung die mindestens eine oxidierende Verbindung enthält gemischt wird, wobei die Zusammensetzung in Teilen entnommen werden kann, ohne die Stabilität des verbleibenden Produkts zu beeinträchtigen.

13. Verwendung einer Verpackung, bestehend aus einer Schraubkappe (A) an einer Flasche die aus einer Dichtung (1), einem Sicherheitsring (2), Gewinde (3) und einem Tragelement (4) besteht und mit einem Silikonventil (5) und einer Arretierung (6) konfektioniert ist, zur stabilen Aufbewahrung eines wässrigen oxidativen Färbemittels welches mindestens einen oxidativen Farbentwickler und optional mindestens eine Kupplerverbindung und optional mindestens eine direkt ziehende Farbe enthält, die vor der Applikation mit einer wässrigen Zusammensetzung die mindestens eine oxidierende Verbindung enthält gemischt wird, wobei die Zusammensetzung in Teilen entnommen werden kann, ohne die Stabilität des verbleibenden Produkts zu beeinträchtigen.

## Revendications

1. Produit multidose pour une coloration des cheveux par oxydation consistant en un emballage consistant en un bouchon à vis (A) sur un tube (7) consistant en une fermeture étanche (1), un indicateur d'effraction (2), un filetage (3) et un élément support (4) et confectionné avec une valve en silicone (5) et un dispositif de maintien pour celle-ci (6) et une composition aqueuse de coloration par oxydation comprenant au moins un précurseur de colorant d'oxydation et facultativement au moins un agent de couplage et facultativement au moins un colorant direct qui est mélangée avec une composition aqueuse comprenant au moins un agent oxydant avant application, dans lequel la composition peut être sortie partiellement sans affecter la stabilité de la composition restante.

2. Produit multidose pour une coloration des cheveux par oxydation consistant en un emballage consistant en un bouchon à vis (A) sur un composant d'emballage en sachet (8) consistant en une fermeture étanche (1), un indicateur d'effraction (2), un filetage (3) et un élément support (4) et confectionné avec une valve en silicone (5) et un dispositif de maintien pour celle-ci (6) et une composition aqueuse de coloration par oxydation comprenant au moins un précurseur de colorant d'oxydation et facultativement au moins un agent de couplage et facultativement au moins un colorant direct qui est mélangée avec une composition aqueuse comprenant au moins un agent oxydant avant application, dans lequel la composition peut être sortie partiellement sans affecter la stabilité de la composition restante.

3. Produit multidose pour une coloration des cheveux par oxydation consistant en un emballage consistant en un bouchon à vis (A) sur une bouteille consistant en une fermeture étanche (1), un indicateur d'effraction (2), un filetage (3) et un élément support (4) et confectionné avec une valve en silicone (5) et un dispositif de maintien pour celle-ci (6) et une composition aqueuse de coloration par oxydation comprenant au moins un précurseur de colorant d'oxydation et facultativement au moins un agent de couplage et facultativement au moins un colorant direct qui est mélangée avec une composition aqueuse comprenant au moins un agent oxydant avant application, dans lequel la composition peut être sortie partiellement sans affecter la stabilité de la composition restante.

4. Produit multidose selon l'une quelconque des revendications précédentes **caractérisé en ce que** la composition colorante aqueuse a une viscosité dans la plage de 1 000 à 50 000 mPa.s mesurée à 20°C avec un viscosimètre Brookfield équipé d'un mobile 5 à 5 tr/min.

5. Produit multidose selon l'une quelconque des revendications précédentes **caractérisé en ce qu'**il comprend au moins une substance de couplage en plus du précurseur de colorant d'oxydation.

6. Produit multidose selon l'une quelconque des revendications précédentes **caractérisé en ce qu'**il comprend au moins un colorant direct choisi parmi des colorants anioniques, cationiques et nitro neutres.

7. Produit multidose selon la revendication 6 en ce que le colorant direct est un colorant capillaire cationique.

8. Produit multidose selon la revendication 6 en ce que le colorant direct est un colorant capillaire nitro neutre.

9. Produit multidose selon l'une quelconque des revendications précédentes **caractérisé en ce que** la composition de coloration par oxydation aqueuse est une émulsion et comprend au moins un agent émulsionnant.

10. Produit multidose selon l'une quelconque des revendications précédentes **caractérisé en ce que** la composition de coloration par oxydation aqueuse a un pH entre 5 et 12.

11. Utilisation d'un emballage consistant en un bouchon à vis (A) sur un tube (7) consistant en une fermeture étanche (1), un indicateur d'effraction (2), un filetage (3) et un élément support (4) et confectionné avec une valve en silicone (5) et un dispositif de maintien pour celle-ci (6) pour stocker de façon stable une composition aqueuse de coloration par oxydation comprenant au moins un précurseur de colorant d'oxydation et facultativement au moins un agent de couplage et facultativement au moins un colorant direct qui est mélangée avec une composition aqueuse comprenant au moins un agent oxydant avant application, dans laquelle la composition peut être sortie partiellement sans affecter la stabilité de la composition restante.

12. Utilisation d'un emballage consistant en un bouchon à vis (A) sur un composant d'emballage en sachet (8) consistant en une fermeture étanche (1), un indicateur d'effraction (2), un filetage (3) et un élément support (4) et confectionné avec une valve en silicone (5) et un dispositif de maintien pour celle-ci (6) pour stocker de façon stable une composition aqueuse de coloration par oxydation comprenant au moins un précurseur de colorant d'oxydation et facultativement au moins un agent de couplage et facultativement au moins un colorant direct qui est mélangée avec une composition aqueuse comprenant au moins un agent oxydant avant application, dans laquelle la composition peut être sortie partiellement sans affecter la stabilité de la composition restante.

13. Utilisation d'un emballage consistant en un bouchon à vis (A) sur une bouteille consistant en une fermeture étanche (1), un indicateur d'effraction (2), un filetage (3) et un élément support (4) et confectionné avec une valve en silicone (5) et un dispositif de maintien pour celle-ci (6) pour stocker de façon stable une composition aqueuse de coloration par oxydation comprenant au moins un précurseur de colorant d'oxydation et facultativement au moins un agent de couplage et facultativement au moins un colorant direct qui est mélangée avec une composition aqueuse comprenant au moins un agent oxydant avant application, dans laquelle la composition peut être sortie partiellement sans affecter la stabilité de la composition restante.
